# EUROPEAN PATENT APPLICATION

(11) **EP 3 011 950 A1**
(43) Date of publication of application: **27.04.2016**
(21) Application number: 14775264.6
(22) Date of filing: 26.03.2014
(51) Int. Cl.: A61K 8/49, A61K 31/37, A61P 39/00

(54) **USE OF THE INHIBITOR OF STEROID SULFATASES STX64 FOR TREATING AGING**

(30) Priority: 26.03.2013 ES 201330436
(71) Applicant: Universidad Pablo de Olavide, 41013 Sevilla (ES)
(72) Inventor: MUÑOZ RUIZ, Manuel Jesús, E-41013 Sevilla (ES); PÉREZ JIMÉNEZ, María Mercedes, E-41013 Sevilla (ES)
(74) Representative: Pons Ariño, Angel
(86) International application number: PCT/ES2014/070230
(87) International publication number: WO 2014/154927

(57) **Abstract**

The invention relates to the use of the inhibitor of the activity of the steroid sulfatase enzyme STX64 for treating aging, and thus for increasing the longevity of individuals or improving their quality of life. The invention also relates to a cosmetic composition comprising said inhibitor for this purpose.

LISTADO DE SECUENCIAS

<110>  Universidad Pablo de Olavide

<120>  "USO DE INHIBIDORES DE SULFATASAS ESTEROIDEAS PARA EL TRATAMIENTO
        DEL ENVEJECIMIENTO"

<130>  ES2762.3

<160>  5

<170>  PatentIn version 3.5

<210>  1
<211>  583
<212>  PRT
<213>  Homo sapiens

<400>  1

Met Pro Leu Arg Lys Met Lys Ile Pro Phe Leu Leu Leu Phe Phe Leu
1               5                   10                  15

Trp Glu Ala Glu Ser His Ala Ala Ser Arg Pro Asn Ile Ile Leu Val
            20                  25                  30

Met Ala Asp Asp Leu Gly Ile Gly Asp Pro Gly Cys Tyr Gly Asn Lys
        35                  40                  45

Thr Ile Arg Thr Pro Asn Ile Asp Arg Leu Ala Ser Gly Gly Val Lys
    50                  55                  60

Leu Thr Gln His Leu Ala Ala Ser Pro Leu Cys Thr Pro Ser Arg Ala
65                  70                  75                  80

Ala Phe Met Thr Gly Arg Tyr Pro Val Arg Ser Gly Met Ala Ser Trp
                85                  90                  95

Ser Arg Thr Gly Val Phe Leu Phe Thr Ala Ser Ser Gly Gly Leu Pro
            100                 105                 110

Thr Asp Glu Ile Thr Phe Ala Lys Leu Leu Lys Asp Gln Gly Tyr Ser
        115                 120                 125

Thr Ala Leu Ile Gly Lys Trp His Leu Gly Met Ser Cys His Ser Lys
    130                 135                 140

Thr Asp Phe Cys His His Pro Leu His His Gly Phe Asn Tyr Phe Tyr
145                 150                 155                 160

Gly Ile Ser Leu Thr Asn Leu Arg Asp Cys Lys Pro Gly Glu Gly Ser
                165                 170                 175

Val Phe Thr Thr Gly Phe Lys Arg Leu Val Phe Leu Pro Leu Gln Ile
            180                 185                 190

Val Gly Val Thr Leu Leu Thr Leu Ala Ala Leu Asn Cys Leu Gly Leu
        195                 200                 205

Leu His Val Pro Leu Gly Val Phe Phe Ser Leu Leu Phe Leu Ala Ala
    210                 215                 220

Leu Ile Leu Thr Leu Phe Leu Gly Phe Leu His Tyr Phe Arg Pro Leu
225                 230                 235                 240

Asn Cys Phe Met Met Arg Asn Tyr Glu Ile Ile Gln Gln Pro Met Ser
                245                 250                 255

Tyr Asp Asn Leu Thr Gln Arg Leu Thr Val Glu Ala Ala Gln Phe Ile
            260                 265                 270

Gln Arg Asn Thr Glu Thr Pro Phe Leu Leu Val Leu Ser Tyr Leu His
        275                 280                 285

Val His Thr Ala Leu Phe Ser Ser Lys Asp Phe Ala Gly Lys Ser Gln
    290                 295                 300

His Gly Val Tyr Gly Asp Ala Val Glu Glu Met Asp Trp Ser Val Gly
305                 310                 315                 320

Gln Ile Leu Asn Leu Leu Asp Glu Leu Arg Leu Ala Asn Asp Thr Leu
                325                 330                 335

Ile Tyr Phe Thr Ser Asp Gln Gly Ala His Val Glu Glu Val Ser Ser
            340                 345                 350

Lys Gly Glu Ile His Gly Gly Ser Asn Gly Ile Tyr Lys Gly Gly Lys
        355                 360                 365

Ala Asn Asn Trp Glu Gly Gly Ile Arg Val Pro Gly Ile Leu Arg Trp
    370                 375                 380

Pro Arg Val Ile Gln Ala Gly Gln Lys Ile Asp Glu Pro Thr Ser Asn
385                 390                 395                 400

Met Asp Ile Phe Pro Thr Val Ala Lys Leu Ala Gly Ala Pro Leu Pro
                405                 410                 415

Glu Asp Arg Ile Ile Asp Gly Arg Asp Leu Met Pro Leu Leu Glu Gly
            420                 425                 430

Lys Ser Gln Arg Ser Asp His Glu Phe Leu Phe His Tyr Cys Asn Ala
        435                 440                 445

Tyr Leu Asn Ala Val Arg Trp His Pro Gln Asn Ser Thr Ser Ile Trp
    450                 455                 460

Lys Ala Phe Phe Phe Thr Pro Asn Phe Asn Pro Val Gly Ser Asn Gly
465                 470                 475                 480

Cys Phe Ala Thr His Val Cys Phe Cys Phe Gly Ser Tyr Val Thr His
                485                 490                 495

His Asp Pro Pro Leu Leu Phe Asp Ile Ser Lys Asp Pro Arg Glu Arg
            500                 505                 510

Asn Pro Leu Thr Pro Ala Ser Glu Pro Arg Phe Tyr Glu Ile Leu Lys
        515                 520                 525

Val Met Gln Glu Ala Ala Asp Arg His Thr Gln Thr Leu Pro Glu Val
    530                 535                 540

Pro Asp Gln Phe Ser Trp Asn Asn Phe Leu Trp Lys Pro Trp Leu Gln
545                 550                 555                 560

Leu Cys Cys Pro Ser Thr Gly Leu Ser Cys Gln Cys Asp Arg Glu Lys
                565                 570                 575

Gln Asp Lys Arg Leu Ser Arg
            580

<210>  2
<211>  1686
<212>  DNA
<213>  Caenorhabditis elegans

<400>  2
atgtccccgg tgagcctatt actacttctt ctattcccat tgagcacttt tcaacaacga      60

gaagattgca cagtgcagcc accatctcca cgtcatccga acattgtgat tcttatgatt     120

gacgacttgg gttacggtga cattgcgtca tacggtcacc cgactcaaga atacacacaa     180

gtcgatcgga tggcagcaga aggcacccgg tttacacaag catattcagc agatagtatg     240

tgctcaccta gcagagctgg attcattact ggacgattac ctattcgatt aggtatagtt     300

ggaggacgga gagtttttgt tccttatgat attggaggcc ttccaaaaag tgagacaaca     360

atggctgaaa tgcttcaaga agcaggatat gcaactggca tggttggaaa atggcatctt     420

ggaattaatg aaaacaatgc aactgatggt gcacatttgc cttcaaagcg aggatttgag     480

tatgttggag tcaacttgcc gtttaccaac gtctggcagt gcgatacaac aagagaattt 540

tacgacaaag gacctgatcc atcactttgt ttcctctatg atggagatga tatcgttcaa 600

caaccaatga aatttgagca tatgacagaa aatcttgttg gagactggaa aaggtttttg 660

atgacaaggt tggcacaaga tcaacacgag agaccattct tcttctattt ctcatttcca 720

caagtccact caacacagtt tgcaagcaaa cgatttagag gatcatcggt tagaggaatc 780

tatggagact ctatcaatga gatgtcatgg gcagttggag aggttttaga cagtcttgta 840

aatgctggaa ttgctgaaaa tacattggtt attttaatgt ctgatcatgg accacatgtt 900

gaactatgtc taaatggtgg atcaacagct ggattgaaag gaggaaagtc gaatagttat 960

gagggaggat tccgaattcc tttcattgct tggcagccag ggacagtgaa accatcccga 1020

gtatcgcatg aagtcatttc cagtatggac ttgtttccaa ctttccgagg aatgcaagaa 1080

caatgcttat ttgagaaaga agcactccgt tctgacggaa ttgatatctc tgacgaattg 1140

cgtggtgaga gtgaagatgt ggaaggaagc ctcggaaagg ctcgtccgat catctactac 1200

tgtaatactc atctgatggc tattaggatg ggagactaca aagttcacta caaaacatct 1260

ccaatcttct ttaataattc agttgatcca aatcttgatt atttctgtcc aaatggaaaa 1320

ccaaagtctg attggtacgt ttcacaagtc tgtcctgatg agcatctaca aaaacattat 1380

cctccacttg ttttcgatct tattcgtgat ccatatgagc agtatccatt gcaaaatact 1440

gtaaaatcac aagagataag attccaagct atgcagcggc tgtctgaaca caaatcgtcg 1500

ctggtcaaag ttaaaaatgt gctcggaagc tacaataaaa cattgatacc ttgctgcaat 1560

ccgccaagtt gcaaatgtga caaattatca aggcccacag aatttgacga aagtcggcca 1620

gattatgttg gtctggtgcc tgatcttgaa aagaccgaat acgaacttct tcacagattc 1680

ttctaa 1686

<210> 3
<211> 561
<212> PRT
<213> Caenorhabditis elegans

<400> 3

Met Ser Pro Val Ser Leu Leu Leu Leu Leu Leu Phe Pro Leu Ser Thr
1               5                   10                  15

Phe Gln Gln Arg Glu Asp Cys Thr Val Gln Pro Pro Ser Pro Arg His
            20                  25                  30

Pro Asn Ile Val Ile Leu Met Ile Asp Asp Leu Gly Tyr Gly Asp Ile
        35                  40                  45

Ala Ser Tyr Gly His Pro Thr Gln Glu Tyr Thr Gln Val Asp Arg Met
    50                  55                  60

Ala Ala Glu Gly Thr Arg Phe Thr Gln Ala Tyr Ser Ala Asp Ser Met
65                  70                  75                  80

Cys Ser Pro Ser Arg Ala Gly Phe Ile Thr Gly Arg Leu Pro Ile Arg
                85                  90                  95

Leu Gly Ile Val Gly Gly Arg Arg Val Phe Val Pro Tyr Asp Ile Gly
            100                 105                 110

Gly Leu Pro Lys Ser Glu Thr Thr Met Ala Glu Met Leu Gln Glu Ala
        115                 120                 125

Gly Tyr Ala Thr Gly Met Val Gly Lys Trp His Leu Gly Ile Asn Glu
    130                 135                 140

Asn Asn Ala Thr Asp Gly Ala His Leu Pro Ser Lys Arg Gly Phe Glu
145                 150                 155                 160

Tyr Val Gly Val Asn Leu Pro Phe Thr Asn Val Trp Gln Cys Asp Thr
                165                 170                 175

Thr Arg Glu Phe Tyr Asp Lys Gly Pro Asp Pro Ser Leu Cys Phe Leu
            180                 185                 190

Tyr Asp Gly Asp Asp Ile Val Gln Gln Pro Met Lys Phe Glu His Met
        195                 200                 205

Thr Glu Asn Leu Val Gly Asp Trp Lys Arg Phe Leu Met Thr Arg Leu
    210                 215                 220

Ala Gln Asp Gln His Glu Arg Pro Phe Phe Phe Tyr Phe Ser Phe Pro
225                 230                 235                 240

Gln Val His Ser Thr Gln Phe Ala Ser Lys Arg Phe Arg Gly Ser Ser
                245                 250                 255

Val Arg Gly Ile Tyr Gly Asp Ser Ile Asn Glu Met Ser Trp Ala Val
            260                 265                 270

Gly Glu Val Leu Asp Ser Leu Val Asn Ala Gly Ile Ala Glu Asn Thr
        275                 280                 285

Leu Val Ile Leu Met Ser Asp His Gly Pro His Val Glu Leu Cys Leu
    290                 295                 300

Asn Gly Gly Ser Thr Ala Gly Leu Lys Gly Gly Lys Ser Asn Ser Tyr
305                 310                 315                 320

Glu Gly Gly Phe Arg Ile Pro Phe Ile Ala Trp Gln Pro Gly Thr Val
                325                 330                 335

Lys Pro Ser Arg Val Ser His Glu Val Ile Ser Ser Met Asp Leu Phe
            340                 345                 350

Pro Thr Phe Arg Gly Met Gln Glu Gln Cys Leu Phe Glu Lys Glu Ala
        355                 360                 365

Leu Arg Ser Asp Gly Ile Asp Ile Ser Asp Glu Leu Arg Gly Glu Ser
    370                 375                 380

Glu Asp Val Glu Gly Ser Leu Gly Lys Ala Arg Pro Ile Ile Tyr Tyr
385                 390                 395                 400

Cys Asn Thr His Leu Met Ala Ile Arg Met Gly Asp Tyr Lys Val His
                405                 410                 415

Tyr Lys Thr Ser Pro Ile Phe Phe Asn Asn Ser Val Asp Pro Asn Leu
            420                 425                 430

Asp Tyr Phe Cys Pro Asn Gly Lys Pro Lys Ser Asp Trp Tyr Val Ser
        435                 440                 445

Gln Val Cys Pro Asp Glu His Leu Gln Lys His Tyr Pro Pro Leu Val
    450                 455                 460

Phe Asp Leu Ile Arg Asp Pro Tyr Glu Gln Tyr Pro Leu Gln Asn Thr
465                 470                 475                 480

Val Lys Ser Gln Glu Ile Arg Phe Gln Ala Met Gln Arg Leu Ser Glu
                485                 490                 495

His Lys Ser Ser Leu Val Lys Val Lys Asn Val Leu Gly Ser Tyr Asn
            500                 505                 510

Lys Thr Leu Ile Pro Cys Cys Asn Pro Pro Ser Cys Lys Cys Asp Lys
        515                 520                 525

Leu Ser Arg Pro Thr Glu Phe Asp Glu Ser Arg Pro Asp Tyr Val Gly
    530                 535                 540

Leu Val Pro Asp Leu Glu Lys Thr Glu Tyr Glu Leu Leu His Arg Phe
545                 550                 555                 560

Phe

<210>  4
<211>  561
<212>  PRT
<213>  Secuencia Artificial

<220>
<223>  Mutante Sul-2 (pv17)

<400>  4

Met Ser Pro Val Ser Leu Leu Leu Leu Leu Leu Phe Pro Leu Ser Thr
1               5                   10                  15

Phe Gln Gln Arg Glu Asp Cys Thr Val Gln Pro Pro Ser Pro Arg His
            20                  25                  30

Pro Asn Ile Val Ile Leu Met Ile Asp Asp Leu Gly Tyr Asp Asp Ile
        35                  40                  45

Ala Ser Tyr Gly His Pro Thr Gln Glu Tyr Thr Gln Val Asp Arg Met
    50                  55                  60

Ala Ala Glu Gly Thr Arg Phe Thr Gln Ala Tyr Ser Ala Asp Ser Met
65                  70                  75                  80

Cys Ser Pro Ser Arg Ala Gly Phe Ile Thr Gly Arg Leu Pro Ile Arg
                85                  90                  95

Leu Gly Ile Val Gly Gly Arg Arg Val Phe Val Pro Tyr Asp Ile Gly
            100                 105                 110

Gly Leu Pro Lys Ser Glu Thr Thr Met Ala Glu Met Leu Gln Glu Ala
        115                 120                 125

Gly Tyr Ala Thr Gly Met Val Gly Lys Trp His Leu Gly Ile Asn Glu
    130                 135                 140

Asn Asn Ala Thr Asp Gly Ala His Leu Pro Ser Lys Arg Gly Phe Glu
145                 150                 155                 160

Tyr Val Gly Val Asn Leu Pro Phe Thr Asn Val Trp Gln Cys Asp Thr
                165                 170                 175

Thr Arg Glu Phe Tyr Asp Lys Gly Pro Asp Pro Ser Leu Cys Phe Leu
            180                 185                 190

Tyr Asp Gly Asp Asp Ile Val Gln Gln Pro Met Lys Phe Glu His Met
        195                 200                 205

Thr Glu Asn Leu Val Gly Asp Trp Lys Arg Phe Leu Met Thr Arg Leu
    210                 215                 220

Ala Gln Asp Gln His Glu Arg Pro Phe Phe Phe Tyr Phe Ser Phe Pro
225                 230                 235                 240

Gln Val His Ser Thr Gln Phe Ala Ser Lys Arg Phe Arg Gly Ser Ser
                245                 250                 255

Val Arg Gly Ile Tyr Gly Asp Ser Ile Asn Glu Met Ser Trp Ala Val
            260                 265                 270

Gly Glu Val Leu Asp Ser Leu Val Asn Ala Gly Ile Ala Glu Asn Thr
        275                 280                 285

Leu Val Ile Leu Met Ser Asp His Gly Pro His Val Glu Leu Cys Leu
    290                 295                 300

Asn Gly Gly Ser Thr Ala Gly Leu Lys Gly Gly Lys Ser Asn Ser Tyr
305                 310                 315                 320

Glu Gly Gly Phe Arg Ile Pro Phe Ile Ala Trp Gln Pro Gly Thr Val
                325                 330                 335

Lys Pro Ser Arg Val Ser His Glu Val Ile Ser Ser Met Asp Leu Phe
            340                 345                 350

Pro Thr Phe Arg Gly Met Gln Glu Gln Cys Leu Phe Glu Lys Glu Ala
        355                 360                 365

Leu Arg Ser Asp Gly Ile Asp Ile Ser Asp Glu Leu Arg Gly Glu Ser
    370                 375                 380

Glu Asp Val Glu Gly Ser Leu Gly Lys Ala Arg Pro Ile Ile Tyr Tyr
385                 390                 395                 400

Cys Asn Thr His Leu Met Ala Ile Arg Met Gly Asp Tyr Lys Val His
                405                 410                 415

Tyr Lys Thr Ser Pro Ile Phe Phe Asn Asn Ser Val Asp Pro Asn Leu
            420                 425                 430

Asp Tyr Phe Cys Pro Asn Gly Lys Pro Lys Ser Asp Trp Tyr Val Ser
        435                 440                 445

Gln Val Cys Pro Asp Glu His Leu Gln Lys His Tyr Pro Pro Leu Val
    450                 455                 460

Phe Asp Leu Ile Arg Asp Pro Tyr Glu Gln Tyr Pro Leu Gln Asn Thr
465                 470                 475                 480

Val Lys Ser Gln Glu Ile Arg Phe Gln Ala Met Gln Arg Leu Ser Glu
                485                 490                 495

His Lys Ser Ser Leu Val Lys Val Lys Asn Val Leu Gly Ser Tyr Asn
            500                 505                 510

Lys Thr Leu Ile Pro Cys Cys Asn Pro Pro Ser Cys Lys Cys Asp Lys
        515                 520                 525

Leu Ser Arg Pro Thr Glu Phe Asp Glu Ser Arg Pro Asp Tyr Val Gly
    530                 535                 540

Leu Val Pro Asp Leu Glu Lys Thr Glu Tyr Glu Leu Leu His Arg Phe
545                 550                 555                 560

Phe

<210>  5
<211>  430
<212>  PRT
<213>  Secuencia Artificial

<220>
<223>  Mutante Sul-2 (gk187)

<400>  5

Met Ser Pro Val Gly Lys Trp His Leu Gly Ile Asn Glu Asn Asn Ala
1               5                   10                  15

Thr Asp Gly Ala His Leu Pro Ser Lys Arg Gly Phe Glu Tyr Val Gly
            20                  25                  30

Val Asn Leu Pro Phe Thr Asn Val Trp Gln Cys Asp Thr Thr Arg Glu
        35                  40                  45

Phe Tyr Asp Lys Gly Pro Asp Pro Ser Leu Cys Phe Leu Tyr Asp Gly
    50                  55                  60

Asp Asp Ile Val Gln Gln Pro Met Lys Phe Glu His Met Thr Glu Asn
65                  70                  75                  80

Leu Val Gly Asp Trp Lys Arg Phe Leu Met Thr Arg Leu Ala Gln Asp
                85                  90                  95

Gln His Glu Arg Pro Phe Phe Phe Tyr Phe Ser Phe Pro Gln Val His
            100                 105                 110

Ser Thr Gln Phe Ala Ser Lys Arg Phe Arg Gly Ser Ser Val Arg Gly
        115                 120                 125

Ile Tyr Gly Asp Ser Ile Asn Glu Met Ser Trp Ala Val Gly Glu Val
    130                 135                 140

Leu Asp Ser Leu Val Asn Ala Gly Ile Ala Glu Asn Thr Leu Val Ile
145                 150                 155                 160

Leu Met Ser Asp His Gly Pro His Val Glu Leu Cys Leu Asn Gly Gly
                165                 170                 175

Ser Thr Ala Gly Leu Lys Gly Gly Lys Ser Asn Ser Tyr Glu Gly Gly
            180                 185                 190

Phe Arg Ile Pro Phe Ile Ala Trp Gln Pro Gly Thr Val Lys Pro Ser
        195                 200                 205

Arg Val Ser His Glu Val Ile Ser Ser Met Asp Leu Phe Pro Thr Phe
    210                 215                 220

Arg Gly Met Gln Glu Gln Cys Leu Phe Glu Lys Glu Ala Leu Arg Ser
225                 230                 235                 240

Asp Gly Ile Asp Ile Ser Asp Glu Leu Arg Gly Glu Ser Glu Asp Val
                245                 250                 255

Glu Gly Ser Leu Gly Lys Ala Arg Pro Ile Ile Tyr Tyr Cys Asn Thr
            260                 265                 270

His Leu Met Ala Ile Arg Met Gly Asp Tyr Lys Val His Tyr Lys Thr
        275                 280                 285

Ser Pro Ile Phe Phe Asn Asn Ser Val Asp Pro Asn Leu Asp Tyr Phe
    290                 295                 300

Cys Pro Asn Gly Lys Pro Lys Ser Asp Trp Tyr Val Ser Gln Val Cys
305                 310                 315                 320

Pro Asp Glu His Leu Gln Lys His Tyr Pro Pro Leu Val Phe Asp Leu
                325                 330                 335

Ile Arg Asp Pro Tyr Glu Gln Tyr Pro Leu Gln Asn Thr Val Lys Ser
            340                 345                 350

Gln Glu Ile Arg Phe Gln Ala Met Gln Arg Leu Ser Glu His Lys Ser
        355                 360                 365

Ser Leu Val Lys Val Lys Asn Val Leu Gly Ser Tyr Asn Lys Thr Leu
    370                 375                 380

Ile Pro Cys Cys Asn Pro Pro Ser Cys Lys Cys Asp Lys Leu Ser Arg
385                 390                 395                 400

Pro Thr Glu Phe Asp Glu Ser Arg Pro Asp Tyr Val Gly Leu Val Pro
                405                 410                 415

Asp Leu Glu Lys Thr Glu Tyr Glu Leu Leu His Arg Phe Phe
            420                 425                 430

## Description

The present invention falls within the fields of medicine, veterinary science and cosmetics, specifically within those compounds and compositions that are useful for treating or delaying the undesirable effects associated with ageing and increasing the longevity of people or animals.

### PRIOR ART

Ageing is a problem that affects all of humanity and, in a society with an ever-increasing life expectancy, there is a growing incidence of ageing-related diseases. As a consequence, for many years delaying the effects of ageing has been a major objective for the pharmaceutical industry.

Currently, there is an increasing interest in the search for compounds that delay ageing, and some of the most promising compounds are resveratrol, berry extracts or tyrosol.

Numerous studies on model organisms have shown that resveratrol, a phytoalexin present in a wide variety of foods, such as grapes and the derivatives thereof, has beneficial effects against diseases such as type II diabetes, cardiovascular diseases and cancer. Due to its antioxidant properties, it has been proposed as an anti-ageing compound. However, trials with humans have not corroborated the expected anti-ageing effect of this compound (Chung et al., 2012, Trends Cell Biol., 22(10):546-554).

There is a growing interest in the nutritional benefits of fruits and vegetables, and their role in the prevention of degenerative diseases, especially in the constituents of certain berries, such as blueberries, which are already being distributed as dietary supplements in concentrated juices and extracts (Krenn et al., 2007, Pharmazie, 62(11):803-812).

Tyrosol is a phenolic compound that is present in wines, both white and red, virgin olive oil, vermouth and beer. Its antioxidant activity has been demonstrated *in vitro,* in humans and in the model organism *Caenorhabditis elegans* (Cañuelo et al., 2012, Mech. Ageing Dev., 133(8):563-74).

On the other hand, steroid sulfatase (STS) regulates the formation of oestrone and dehydroepiandrosterone (DHEA), which are capable of promoting tumour growth, by the desulfation of the corresponding sulfate conjugates. The use of steroid sulfatase inhibitors is an increasingly-used process due to its potential therapeutic effect as an anti-tumour drug for various types of hormone-dependent cancers, such as breast cancer, prostate cancer, uterine cancer, endometrial cancer or thyroid cancer (EP1568381A1). The irreversible steroid sulfatase inhibitor known as 667 COUMATE, STX 64, BN83495 or lrosustat has been the first compound of this type to proceed to the clinical trial stage as a therapy for steroid-hormone-dependent cancers (Purohit & Foster, 2012, J. Endocrinol., 212(2):99-110).

Moreover, different steroid sulfatase inhibitors are also receiving close attention due to their potential therapeutic effect for the treatment of endometriosis, infertility, autoimmune diseases, dementia or Alzheimer's disease (EP1193250A1). They have also been proposed to control immunological and inflammatory responses (EP0758230B1).

Therefore, currently there are no compounds that are useful for the effective treatment of ageing. As a consequence, there is a need to search for new pharmacological targets with a clinical or cosmetic relevance that make it possible to develop compounds which may be used for the treatment of the adverse effects associated with ageing and, thus, for delaying it.

### DESCRIPTION OF THE INVENTION

The present invention proposes the use of inhibitors of the activity of the steroid sulfatase enzyme (STS) for the treatment of ageing. The examples shown further below demonstrate that the total or partial inhibition of the activity of this enzyme entails a delay in ageing and, therefore, a significant increase in the longevity of the individuals treated as compared to the control subjects.

In the absence of effective therapeutic or cosmetic strategies for the pharmacological treatment of ageing and its associated effects, the present invention provides a solution to the problem by treating ageing using inhibitors of the activity of the enzyme steroid sulfatase enzyme.

For this reason, a first aspect of the invention relates to the use of at least one inhibitor of the activity of the steroid sulfatase enzyme for the treatment of ageing or its associated effects in humans and/or animals, or alternatively, to the use of at least one inhibitor of the activity of the steroid sulfatase enzyme for the preparation of a composition designed for the treatment of ageing or its associated effects in humans and/or animals. Hereinafter, we will use the terms "use of the present invention" or "use of the invention" to refer to the use described in this paragraph.

The "steroid sulfatase enzyme", "STS", "steryl-sulfatase", "arylsulfatase", "ASC" or "steryl-sulfatase sulfohydrolase", is the microsomal enzyme that splits off sulfate groups from the inactive forms (sulfated) of several steroid hormones. This steroid desulfation plays a significant role in several physiological processes, such as the maturation of epidermal cells, pregnancy and the immune response. This enzyme is present in a variety of organisms, such as, for example, *Homo sapiens, Pongo abelii, Nomascus leucogenys, Pan troglodytes, Macaca mulatta, Macaca fascicularis, Callithrix jacchus, C. elegans, Loxodonta africana, Ailuropoda melanoleuca, Canis lupus familiaris, Equus caballus, Felis catus, Cavia porcellus,* etc. In a preferred embodiment, the steroid sulfatase enzyme to which the present invention relates is the enzyme with the amino acid sequence SEQ ID NO: 3, or enzyme Sul-2 from *C*. *elegans,* which is encoded by the nucleotide sequence SEQ ID NO: 2. In another preferred embodiment, the steroid sulfatase enzyme to which the present invention relates is the human enzyme EC 3.1.6.2, with 583 amino acids and SEQ ID NO: 1.

The present invention clearly and unequivocally demonstrates that the specific inhibition of the activity of the STS enzyme increases the life expectancy of those individuals who have been administered with inhibitors of said activity. Therefore, the examples of the present invention demonstrate that specific chemical antagonists against the STS enzyme, as well as loss-of-function mutations in the gene that encodes the enzyme, solve the technical problem of the present invention, and these examples are a clear demonstration that inhibitors of a very different nature have the same technical effect; consequently, the present invention should not be limited to the use of the specific inhibitors assayed, but should also relate to those inhibitors that inhibit, partially or totally, the activity of the enzyme which are known at the time of filing of the present invention. In sum, the present invention contributes to the prior art the evidence that the inhibition of said enzymatic activity, through any means or product, is useful for the treatment of ageing.

The term "inhibitor of the activity of the steroid sulfatase enzyme", as used in the present invention, refers to a molecule that binds to any of the following elements: the gene that encodes the steroid sulfatase enzyme, transcription factors of said gene, any of the expression products of said gene, for example, without being limited thereto, the messenger RNA or the steroid sulfatase enzyme, and decreases or inhibits the expression and the activity of the molecule to which it binds, and/or its intracellular signalling, thereby leading to total or partial inhibition of the activity of the steroid sulfatase enzyme. In another preferred embodiment, said inhibitor is selected from the list consisting of, without being limited thereto: antagonists against the steroid sulfatase enzyme (preferably chemical), silencing RNA or specific antibody against the steroid sulfatase enzyme (preferably, the antibody is monoclonal); in the present invention, this antibody may be defined as a neutralising antibody against the effect of the steroid sulfatase enzyme. Examples of chemical inhibitors of the activity of the steroid sulfatase enzyme are, without being limited thereto, alternative substrates such as those in the series 2-(hydroxyphenyl) indol sulfate, synthetic or natural steroids which present inhibitory activity against STS, such as 5-androstene-3β, 17β-diol-3 sulfate, competitive inhibitors such as E₁-MTP or EMATE, non-oestrogenic inhibitors such as COUMATE or STX64, or others, such as KW-2581 or STX213, whose IC50 against the STS enzyme has been determined in different studies (Purohit & Foster, 2012, J. Endocrinol., 212(2):99-110). For this reason, in a preferred embodiment, the inhibitor of the activity of the steroid sulfatase enzyme is selected from the list consisting of: 2-(hydroxyphenyl) indol sulfate, 5-androstene-3β, 17β-diol-3 sulfate, E₁-MTP, EMATE, COUMATE or STX64, KW-2581 and STX213, morpholine, silencing RNA and specific antibody against the steroid sulfatase enzyme.

In a more preferred embodiment, the inhibitor of the activity of the steroid sulfatase enzyme to which the present invention relates is STX 64.

The inhibitor "STX 64" or "667 COUMATE", "BN83495" or "Irosustat" is the compound with the number CAS 288628-05-7, the formula C₁₄H₁₅NO₅S and formula (I):

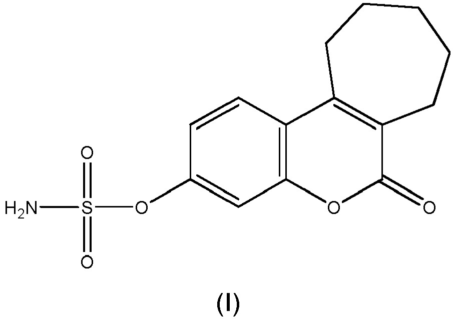

The inhibitors of the present invention may exist in the form of enantiomers or diastereomers. The present invention also considers the use of solvates of the antagonist (such as, for example, without being limited thereto, hydrates), prodrugs, or clathrates. The pharmaceutically acceptable salts are selected from chloride, bromide, iodide or any other pharmaceutically acceptable salt.

The antagonists against the activity of the steroid sulfatase enzyme of the present invention may include isomers, depending upon the presence of multiple bonds (for example, Z, E), including optical isomers, or enantiomers, depending upon the presence of chiral centres. The individual isomers, enantiomers or diastereoisomers and the mixtures thereof fall within the scope of the present invention. The individual enantiomers or diastereoisomers, as well as the mixtures thereof, may be separated by means of conventional techniques.

In the present invention, the term " pharmaceutically acceptable salts" of the antagonist compounds refers to salts prepared from non-toxic, pharmaceutically acceptable acids, including organic and inorganic acids. The non-toxic organic or inorganic acids are selected from the list comprising, without being limited thereto: acetic acid, alginic acid, anthranilic acid, benzenesulfonic acid, benzoic acid, camphorsulfonic acid, citric acid, ethanesulfonic acid, formic acid, fumaric acid, furoic acid, gluconic acid, glutamic acid, glucorenic acid, galacturonic acid, glycidic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phenyl acetic acid, propionic acid, phosphoric acid, salicylic acid, stearic acid, succinic acid, sulfanilic acid, sulfuric acid, tartaric acid or p-toluenesulfonic acid.

The inhibitors to which the invention relates may be in crystalline form, as free compounds or solvates. In this sense, the term "solvate", as used herein, includes both pharmaceutically and pharmacologically acceptable solvates, i.e. solvates of the antagonist against the activity of the steroid sulfatase enzyme, which may be used in the preparation of a composition, preferably of a medicament, and non-pharmaceutically acceptable solvates, which may be used in the preparation of pharmaceutically and pharmacologically acceptable solvates or salts. The nature of the pharmaceutically acceptable solvate is not critical, provided that it is pharmaceutically acceptable. In a particular embodiment, the solvate is a hydrate. The solvates may be obtained by means of conventional solvation methods known to persons skilled in the art.

Moreover, the scope of this invention also includes prodrugs of the antagonists against the activity of the steroid sulfatase enzyme. The term "prodrug", as used herein, includes any derivative compound of the antagonist, for example, without being limited thereto: esters (including carboxylic acid esters, amino acid esters, phosphate esters, metal salt sulfonate esters, etc.), carbamates, amides, biohydrolysable amides, biohydrolysable esters, biohydrolysable carbamates, biohydrolysable ureides and biohydrolysable phosphates. Other examples of prodrugs include compounds that comprise - NO, -NO₂, -ONO, or -ONO₂- groups, which, upon being administered to an individual, may be converted, directly or indirectly, into said antagonist in said individual. Advantageously, said derivative is a compound that increases the bioavailability of the antagonist when administered to an individual or which enhances the release of the antagonist in a biological compartment. The nature of said derivative is not critical, provided that it may be administered to an individual and supplies the antagonist against the activity of the steroid sulfatase enzyme in a biological compartment thereof. The preparation of said prodrug may be performed by means of conventional methods known to persons skilled in the art. The terms "biohydrolysable amides", "biohydrolysable esters", "biohydrolysable carbamates", "biohydrolysable ureides" and "biohydrolysable phosphates" refer to the carbamates, carbonates, ureides and phosphates, respectively, of a compound which: 1) do not interfere with the biological activity of the complex, but provide the compound with advantageous properties *in vivo,* such as absorption, duration of effect, or onset of effect; or 2) are biologically inactive, but become biologically active compounds *in vivo.*

Other inhibitors to which the invention relates are those that hinder or inhibit the production of the steroid sulfatase enzyme by affecting the expression, or/and the translation or/and the stability of RNA, such as morpholines or antisense or silencing RNAs. In regards to silencing RNAs, nucleotides (tt) or (tg) may be added to the 3' end of the sense chain or to the 5' end of the antisense chain of the siRNA that acts as an inhibitor of the activity of the steroid sulfatase enzyme, in order to improve their function. Therefore, these are overhanging nucleotides, which do not hybridise with any other nucleotide in the complementary chain. The addition of these nucleotides at the 3' or 5' end does not affect the recognition of the corresponding messenger RNA.

The term "treatment", as understood in the present invention, refers to fighting the ageing-related adverse effects in a subject (animal and, preferably, human), and includes:
(i) delaying the process of ageing; and/or
(ii) alleviating the associated effects of ageing.

The term "ageing", or "senescence", refers to the set of morphological and physiological modifications that appear as a consequence of the action of time on living beings, preferably humans, which entail a decrease in the adaptive capacity of all the organs and systems, as well as in the capacity to respond to external stimuli and agents that affect the individual. The main ageing-associated symptoms or effects are, without being limited thereto, progressive loss of visual capacity, such as presbyopia, myopia, cataracts, etc., progressive hypoaccusia, loss of muscle elasticity, sleep alterations, loss of reflex reaction agility and capacity, degeneration of bone structures, which is generally associated with the appearance of deformations caused by acromegalies, osteoporosis, rheumatoid arthritis, etc., senile dementia, such as, for example, Alzheimer's disease, distension of supporting muscle tissues, progressive loss of muscular strength, increase in high blood pressure, prostate alterations, loss of the immune capacity against infectious agents, decrease in skin collagen and protein absorption capacity, progressive loss of the taste sensation and progressive loss of libido, decrease in spermatogenesis in men and menopause in women. The inhibitors of the activity of the steroid sulfatase enzyme proposed in the present invention are useful to alleviate and/or delay these effects associated with ageing.

The term "composition" refers to any cosmetic or medicinal preparation. The composition to which the present invention relates may comprise one or several inhibitors of the activity of the steroid sulfatase enzyme, preferably those described in the present invention, in any mixture thereof.

In another preferred embodiment, the composition to which the present invention relates is a cosmetic composition.

In another preferred embodiment, the composition to which the present invention relates is a medicament. The medicament to which the present invention relates may be for human or veterinary use. A "medicament for human use" is any substance or combination of substances which has properties indicated for the treatment of ageing or the associated effects thereof in human beings or which may be used in human beings or be administered to human beings in order to restore, correct or modify physiological functions by exerting a pharmacological, immunological or metabolic action. A "medicament for veterinary use" is any substance or combination of substances which has properties indicated for the treatment of ageing or the associated effects thereof in non-human animals or which may be administered to animals in order to restore, correct or modify their physiological functions by exerting a pharmacological, immunological or metabolic action. Also considered as "veterinary medicaments" are "pre-mixes for medicated feedingstuffs" prepared to be incorporated into feedingstuffs.

In a more preferred embodiment, the composition to which the present invention relates further comprises a pharmaceutically acceptable vehicle and/or another active principle. Said composition may further comprise an excipient.

The term "excipient" refers to a substance that helps in the absorption of any of the components of the composition to which the present invention relates, stabilises said components or helps in the preparation of the composition in the sense of providing it with consistency or supplying flavours that make it more pleasant. Therefore, excipients may serve as binding agents for the components, such as, for example starches, sugars or celluloses; sweetening agents; colouring agents; protective agents for the medicament, designed, for example, to isolate it from the air and/or humidity; filler agents for pills, capsules or any other form of presentation, such as, for example, dibasic calcium phosphate; disintegrating agents, designed to facilitate dissolution of the components and their absorption in the intestine; without excluding other types of excipients not mentioned in this paragraph. Therefore, the term "excipient" is defined as that matter which, when included in "galenic forms", is added to the active principles or the associations thereof in order to facilitate the preparation and stability of the composition, modify its organoleptic properties or determine its physico-chemical properties and bioavailability.

The "galenic form or pharmaceutical form" is the arrangement of the active principles and the excipients to compose a medicament. It is defined by the combination of the form under which the composition is presented by the manufacturer and the form in which it is administered.

Preferably, the "pharmaceutically acceptable vehicle", or "carrier", is an inert substance. The function of the vehicle is to facilitate the incorporation of other compounds, allow for a better dosage and administration, or provide the composition with consistency and shape. Therefore, the vehicle is a substance that is used in the composition to dilute any of the components thereof to a given volume or weight, or, even without diluting said components, is capable of allowing for a better dosage and administration, or providing the composition with consistency and shape. When the form of presentation is liquid, the pharmaceutically acceptable vehicle is the diluent.

Moreover, the excipient and the vehicle must be pharmacologically acceptable, i.e. the excipient and the vehicle must be authorised and evaluated such that they do not cause any harm to the organisms to whom they are administered.

On the other hand, optionally, the composition described may further comprise another active substance. In addition to the need for therapeutic efficacy, which may require the use of other therapeutic agents in said composition, there may be additional important reasons that force to or largely recommend the use of a combination of at least one inhibitor of the activity of the steroid sulfatase enzyme and another therapeutic agent. The term "active principle" is any matter, whatever its origin, human, animal, vegetable, chemical or of another type, that is considered to have an appropriate activity to compose a medicament. In a more preferred embodiment, the other active principle included in the composition is a compound designed for the treatment of ageing. Examples of compounds designed for the treatment of ageing are, without being limited thereto, polyphenols, such as resveratrol, or, in general, any compound with antioxidant properties.

Preferably, the composition of the invention comprises a steroid sulfatase inhibitor in a therapeutically effective quantity, where "therapeutically effective quantity" is understood to mean the level, quantity or concentration of said inhibitor that produces the desired effect without causing adverse effects. The dosage required to obtain a therapeutically effective quantity is dependent on a variety of factors, such as, for example, the age, weight, sex or tolerance of the individual to whom the pharmaceutical composition of the invention is to be administered.

The composition of the present invention may be formulated to be administered in a variety of forms known in the prior art. Examples of preparations include solid compositions (tablets, pills, capsules, granules, etc.) or liquid compositions (solutions, suspensions or emulsions) for oral, topical or parenteral administration. The pharmaceutical composition of the present invention may also be in the form of sustained-release drug formulations or any other conventional release system; thus, it may be contained, without being limited thereto, in nanoparticles, liposomes or nanospheres, in a polymeric material, in a biodegradable or non-biodegradable implant, or in biodegradable microparticles, such as, for example, biodegradable microspheres.

Such composition and/or the formulations thereof may be administered to an animal, including a mammal and, therefore, a human being, in a variety of forms, including, without being limited thereto, parenteral, intraperitoneal, intravenous, intradermal, epidural, intraspinal, intrastromal, intra-articular, intrasynovial, intrathecal, intralesional, intra-arterial, intracardiac, intramuscular, intranasal, intracraneal, subcutaneous, intraorbital, intracapsular or topical, by means of transdermal patches, by vaginal route or rectal route, through the administration of suppositories, by percutaneous route, or by means of nasal sprays, surgical implants, internal surgical paint, infusion pumps or catheters.

In each case, the form of presentation of the composition will be adapted to the type of administration used; to this end, the composition to which the present invention relates may be presented in the form of solutions or any other clinically permitted form of administration, in a therapeutically effective quantity. The composition may be formulated in solid, semi-solid, liquid or gas forms, such as pills, capsules, powders, granules, ointments, solutions, suppositories, injections, inhalants, gels, microspheres or aerosols. The composition may be presented in a form that is adapted to, for example, without being limited thereto, oral, sublingual, nasal, intrathecal, bronchial, lymphatic, rectal, vaginal, transdermal, inhalation, topical or parenteral administration. Preferably, the composition is formulated for oral, topical or parenteral administration. On the other hand, the inhibitor of the activity of the steroid sulfatase enzyme may be associated, for example, without being limited thereto, to liposomes or micelles.

Another aspect of the invention relates to a cosmetic composition that comprises one inhibitor of the activity of the steroid sulfatase enzyme selected from the list consisting of: 2-(hydroxyphenyl) indol sulfate, 5-androstene-3β, 17β-diol-3 sulfate, E₁-MTP, EMATE, COUMATE or STX64, KW-2581, STX213, morpholine, silencing RNA, and specific antibody against the steroid sulfatase enzyme.

Throughout the description and the claims, the word "comprises" and variants thereof are not intended to exclude other technical characteristics, additives, components or steps. For persons skilled in the art, other objects, advantages and characteristics of the invention will arise, partly from the description and partly from the practice of the invention. The following examples and figures are provided for illustrative purposes, and are not intended to limit the scope of the present invention.

### DESCRIPTION OF THE FIGURES

Figure 1 shows the longevity curve at 25°C. In the graph, we may observe the mean life of a population of wild type *C*. *elegans* and a mutant population *sul-2(gk187),* which present a significant difference, with p < 0.0001, n > 100.
Figure 2 shows the longevity curve at 25°C. In the graph, we may observe the mean life of a population of *C*. *elegans* mutant *sul-2(pv17)* and a population of wild type *C*. *elegans,* which present a significant difference, with p < 0.0001, n > 100.
Figure 3 shows the longevity curves for a wild type population supplemented with 0.5 µg/ml or 1 µg/ml of STX64 at 25°C. In the presence of the steroid sulfatase inhibitor STX64, the population lives significantly longer, in both cases, than the control population exposed to the inhibitor solvent (DMSO), p = 0.0139, n > 100.

### EXAMPLES

Below we will illustrate the invention by means of assays performed by the inventors, which demonstrate the efficacy of the use of inhibitors of the activity of the steroid sulfatase enzyme in the treatment and/or prevention of ageing and, therefore, in increased longevity of the individuals treated. These specific examples are provided to illustrate the nature of the present invention and are included solely for illustrative purposes, for this reason they should not be interpreted as limitations to the invention claimed herein. Therefore, the examples described further below illustrate the invention without limiting the scope of application thereof.

### Example 1. The reduction in the activity of the steroid sulfatase enzyme delays ageing.

As will be shown in this example, in the model organism *C*. *elegans* we observed that the reduction in the activity of the steroid sulfatase enzyme by means of genetic mutation delays ageing.

In order to generate the longevity curves, the strains under study and their pertinent controls were first subjected to an egg preparation in order to prevent contaminations and synchronise them at the embryonic stage, and, subsequently, they were incubated at 16°C. In order to prevent interferences caused by physiological stresses resulting from this process, they were allowed to grow to the following generation. 5 or 6 L4 per plate were then transferred to 4 plates, and were once again incubated at 16°C to L4, the stage selected to start the longevity curves.

On day zero, 120 L4 of each strain were transferred to new plates, 20 worms per plate, and incubated at the temperature at which the assay was subsequently performed, i.e. 25°C.

During the first week of the curves, the nematodes were transferred to new plates every 2 days, in order to prevent confusions with potential descendants. Subsequently, they were checked every 2 or 3 days, and those specimens that were lost or dead for non-physiological reasons were withdrawn from the study. The individuals were transferred to new plates every 5 days in order to maintain optimal growth conditions in the plates. The specimens were considered to be dead when they ceased to respond upon being softly touched with the platinum wire.

The longevity was studied in the presence of the steroid sulfatase inhibitor STX64 (SIGMA, S1950-5MG) diluted in DMSO at 5µg/µl. The plates used had a 35-mm diameter and a constant volume of NGM medium of 2 ml; the addition of the inhibitor was performed a couple of hours before placing the nematodes therein, at a concentration of 1 µg/ml of final volume. The preparation and synchronisation of the nematodes until day = 0 was the same as in the preceding case, the only difference being that the nematodes were transferred to fresh plates every two or three days until the curve was completed.

We used the GraphPad Prism 5 programme (Version 5.0a, for Mac OS X. 1992-2008 GraphPad Software, Inc.) in the survival curve mode to represent the longevity curves, and the Kaplan-Meier method was used to perform the statistical analysis thereof. In the figures of the present invention, the standard error of the mean, SEM, of each point is represented by means of error bars and, in order to determine whether there were differences between the curves, the Log-Rank test was applied.

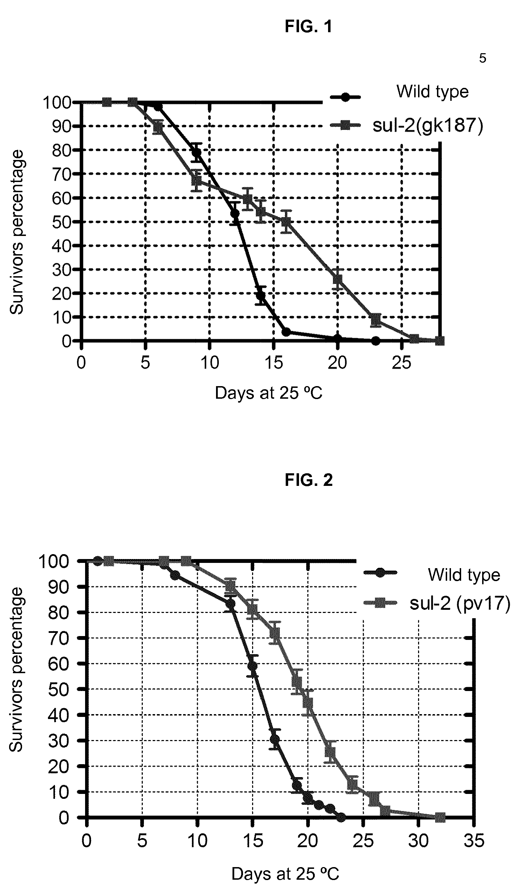

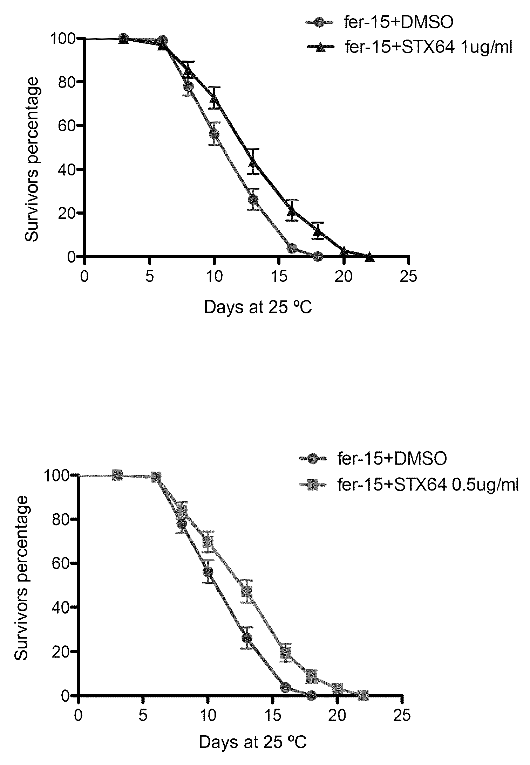

In the first place, mutants of the gene *sul-2* of *C*. *elegans* were generated. The native nucleotide sequence of said gene is SEQ ID NO: 2. Said gene encodes a protein with SEQ ID NO: 3, homologous to the steroid sulfatase of mammals. Those organisms which expressed the mutant protein showed a significant increase in longevity as compared to the wild type organisms. It was demonstrated that both the deletion of the gene *sul-2(gk187),* which results in a protein with SEQ ID NO: 5, and the isolated *pv17* allele, which results in a protein with SEQ ID NO: 4, a loss-of-function mutant, led to an increase in longevity (Figures 1 and 2). And not only that: these mutants furthermore maintained their motor functions even after a large part of the wild type population had died.

This indicates that the inhibition of the function of steroid sulfatases may be used as an anti-ageing treatment. For this reason, an assay was subsequently performed on *C*. *elegans* with the steroid sulfatase inhibitor STX 64, and it was found that the treatment delayed ageing (Figure 3).

## Claims

1. Use of the inhibitor of the activity of the steroid sulfatase enzyme STX64 for the preparation of a composition designed for the treatment of ageing.

2. Use according to claim 1, wherein the steroid sulfatase enzyme is the enzyme with SEQ ID NO: 1.

3. Use according to any of claims 1 to 2, wherein the composition is a cosmetic composition.

4. Use according to any of claims 1 to 2, wherein the composition is a medicament.

5. Use according to any of claims 1 to 4, wherein the composition further comprises another active principle.

6. Use according to claim 5, wherein the other active principle is a compound designed for the treatment of ageing.

7. Use according to any of claims 1 to 6, wherein the composition is formulated for oral, topical or parenteral administration.

8. Cosmetic composition which comprises the inhibitor of the activity of the steroid sulfatase enzyme STX64.

9. Cosmetic composition which comprises an inhibitor of the activity of the steroid sulfatase enzyme selected from the list consisting of: 2-(hydroxyphenyl) indol sulfate, 5-androstene-3β, 17β-diol-3 sulfate, E₁-MTP, EMATE, STX64, KW-2581, STX213, morpholine, silencing RNA, and specific antibody against the steroid sulfatase enzyme.
